# EUROPÄISCHE PATENTSCHRIFT

(11) **EP 0 634 149 B1**
(45) Veröffentlichungstag und Bekanntmachung des Hinweises auf die Patenterteilung: **03.12.2003**
(21) Anmeldenummer: 94810372.6
(22) Anmeldetag: 23.06.1994
(51) Int. Cl.: A61C 13/00, A61K 6/06, A61L 27/00, C04B 35/486, A61F 2/30

(54) **Verfahren zur Herstellung von Prothesen**
Process for manufacturing prostheses
Procédé de fabrication de prothèses

(30) Priorität: 24.06.1993 CH 189593
(43) Veröffentlichungstag der Anmeldung: 18.01.1995
(73) Patentinhaber: METOXIT AG, CH-8240 Thayngen (CH)
(72) Erfinder: Rieger, Wolfhart, Dr., CH-8263 Buch (SH) (CH)
(74) Vertreter: Peege, Klaus

(56) Entgegenhaltungen:
- EP-A- 0 218 853
- EP-A- 0 311 214
- EP-A- 0 455 855
- DE-U- 8 708 806
- US-A- 4 663 720
- DATABASE WPI Section Ch, Week 8718, Derwent Publications Ltd., London, GB; Class D21, AN 87-125072 & JP-A-62 065 972 (SHINAGAWA FIRE BRICK) 25. März 1987

## Beschreibung

Die Erfindung betrifft ein Verfahren zur Herstellung hochpräziser und dreidimensional komplexer keramischer Endo- und Exoprothesen.

Zur Herstellung von Prothesen (Endoprothesen, Exoprothesen) sind metallische und keramische Werkstoffe mit speziell auf diese Materialien abgestimmten Bearbeitungsverfahren bekannt. Zur Herstellung kompliziert gestalteter Prothesen hoher Form-, Mass-, und Passgenauigkeit überwiegt die Verwendung von Metall in Verbindung mit aus der Metallverarbeitung bekannten Bearbeitungsverfahren. Durchgesetzt haben sich für metallische Prothesen Titan mit seinen Legierungen und Chrom/Kobaltstähle. Der Verwendungsvorzug von Metallen der genannten Art leitet sich daraus ab, dass Prothesen maschinell mit engsten Toleranzen bezüglich Form, Dimensionierung und Passgenauigkeit herstellbar sind. Metalle haben den Nachteil, dass ihre Eigenschaften für Endo- und Exoprothesen nicht ganz befriedigen. Chrom/Kobaltstähle zeichnen sich durch hohe Festigkeiten aus, während sie anfällig auf ph-Wertschwankungen von Körperflüssigkeiten sind. Bei Titanlegierungen verhält es sich genau umgekehrt.

Hohe Festigkeiten und chemische Beständigkeit werden mit keramischen Werkstoffen erreicht, hingegen ist die Herstellung keramischer Prothesen deutlich schwieriger als die Herstellung metallischer Prothesen, so dass sich der Einsatz keramischer Werkstoffe auf lasttragende Prothesen unkomplizierter Form (Gelenkkugeln) ohne Anpassungskontourierungen mit einfachen Bearbeitungsverfahren nach Dichtsinterung (Kugelschleifen und Polieren) beschränkt hat. Der Bereich hochpräziser dreidimensional komplex und auch patientenindividuell geformter keramischer Prothesen, z.B. Finger-, Knie- oder Rückgratteilprothesen ist der keramischen Prothetik weitgehend verschlossen. Dies liegt an der unumgänglichen Dichtsinterung oder Infiltration von Rohlingen, damit diese bei hohen Festigkeitswerten chemisch beständig werden und ferner weitere Eigenschaften annehmen, die sie als Rohling im porösen, d.h. nicht gesinterten Zustand nicht aufweisen. Dichtsinterung oder Infiltration verändert in porösem Zustand vorgefertigte Konturen und Masse, so dass nach der Sinterung Nacharbeiten vorzunehmen sind, um eine Prothese auf geforderte Mass- und Formgenauigkeiten zurückzuführen. Die Nacharbeit ist auf Grund der nach der Sinter- oder Infiltrierung vorliegenden Härte sehr schwierig. Hinzu tritt, dass von keramischen Prothesematerialien Eigenschaften gefordert werden, die nicht von allen, sondern nur einigen wenigen Keramiken in relativ engen Zusammensetzungsbereichen erfüllt werden. Endoprothesen (z.B. Finger-, Knie-, Rückgratteilprothesen, etc.) aus keramischen Werkstoffen - auf Endoprothesen dieser Art stellt neben vergleichbaren Exoprothesen die Erfindung ab - sind nur dann als Prothesen verwendbar, wenn ihr Werkstoff physiologisch unbedenklich, die Keramik bioinert, also beständig gegen Körperflüssigkeiten und biokompatibel, d.h. körperverträglich ist. Weiter gefordert ist zur Vermeidung der Aufnahme von Körperflüssigkeit eine gute Dichtsinterbarkeit, in dichtgesintertem Zustand hohe Festigkeiten und Abriebsbeständigkeit. Diese Anforderungen haben keramische Werkstoffe gesamthaft zu erfüllen, ansonsten sie für die keramische Prothetik für beide der hier angesprochenen Bereiche ausser Betracht fallen.

Für grössere lasttragende Endo- und Exoprothesen, z.B. Gelenkkugeln, haben sich zwei keramische Werkstoffe qualifiziert, nämlich Aluminiumoxid (AL203) mit einem AL203 Anteil von 99,85%, Rest andere Bestandteile und Zirkonoxid (Zr02) in überwiegend tetragonaler Struktur, stabilisiert durch Magnesiumoxid (Mg0) oder durch ein Oxid der seltenen Erden, vorzugsweise Yttriumoxid (Y203) oder Ceroxid (Ce02). Für körperlich kompliziert ausgebildete kleine oder kleinste Prothesen sehr hoher Mass- und Formgenauigkeit sind die genannten keramischen Werkstoffe zufolge der Dichtsinterung mit daraus resultierenden Nacharbeitungsschwierigkeiten nach Meinung der Fachwelt nicht geeignet, so dass für Prothesen der vorstehend genannten Art Metalle zu ihrer Herstellung dominieren.

Hiervon ausgehend hat sich der Erfinder die Aufgabe gestellt, ein Verfahren zur Herstellung keramischer Prothesen zu schaffen, mit dem Prothesen gleicher Konturenvielfalt, Form-, Mass- und Passgenauigkeit wie metallische Prothesen herstellbar sind, wobei diese Prothesen die an sie für ihren bestimmungsgemässen Gebrauch zu fordernden Materialeigenschaften aufzuweisen haben und diese Aufgabe wird erfindungsgemäss durch die kennzeichnenden Merkmale des Patentanspruches 1 gelöst.

Vorteilhafte Ausgestaltungen des erfindungsgemässen Verfahrens sind in den dem Anspruch 1 folgenden Ansprüchen gekennzeichnet.

Folgend wird zum besseren Verständnis der Erfindung "Rohling" für ungesintertes oder nicht infiltriertes Material, "Halbzeug" für durch Sichterung, Infiltration oder andere thermische Verfahren aus dem "Rohling" gewonnenes Material, "Prothese" für aus "Halbzeug" durch Umarbeitung vermittels bewegbarer Bearbeitungseinrichtungen und mit bewegten Werkzeugen hergestellte dreidimensional komplexe keramische Teile hoher Form-, Mass- und Passgenauigkeit verwendet.

Ueberraschenderweise wurde gefunden, dass es mit den erfindungsgemässen Verfahrens-, d.h. Bearbeitungsparametern möglich wird, dichtgesinterte keramische, d.h. erfindungsgemäss aus einem Zirkonoxid bestehende Prothesen gleicher Form- und Massgenauigkeit wie metallische Prothesen herzustellen. Auf Grund der grossen Härte dichtgesinterten Zirkonoxides stand nicht zu erwarten, dass mit den erfindungsgemässen Verfahrensparametern geforderte Form- und Massgenauigkeiten erreicht würden. Das Zirkonoxid nach der Erfindung ist bioinert, biokompatibel und erfüllt alle weiteren an keramische Prothesenwerkstoffe zu stellenden Anforderungen, so dass mit der Erfindung der Eingang in den Teil der Prothetik, der bisan vorwiegend Metallen als Prothesewerkstoffen vorbehalten war, mit den daraus resultierenden Vorteilen erreicht ist.

Nach der Erfindung besteht die Möglichkeit zur Herstellung einer Prothese von einem dichtgesinterten oder infiltrierten Halbzeug, beispielsweise einer Ronde aus Zirkonoxid auszugehen, indem die Prothese, obwohl Zirkonoxid in dichtgesintertem Zustand wesentlich schwieriger maschinell zu bearbeiten ist als Aluminiumoxid, und Aluminiumoxid sich somit für die Prothetik der hier angesprochenen Art anbieten würde, aus diesem Halbzeug nach einer Vorlage maschinell herausgearbeitet wird. Auch kann nach der Erfindung zunächst von einem porösen Rohling ausgegangen, dieser unter Dimensionszugaben umgearbeitet, mit Masszugaben als Halbzeug dichtgesintert oder infiltriert und anschliessend vermittels des erfindungsgemässen Verfahrens auf die Endform und -masse einer Prothese bearbeitet werden.

Im Rahmen der Erfindung liegt auch, mit Hilfe einer Form und Schlickergiessen ein keramisches Rohteil herzustellen, dieses zu trocknen, anschliessend zu brennen und heissisostatisch nachzuverdichten und oxidierend nachzubehandeln, anschliessend kann das einer Prothese entsprechende figürliche Halbzeug gemäss der Erfindung fertig bearbeitet werden.

Im folgenden wird die Erfindung an Hand von Beispielen näher erläutert.

### Beispiel I: Fingergelenk

Ein Kunststoffmodell eines Einsatzes für ein Fingergelenk (Proximale Phalanx) wird mit Hilfe einer 3 D-Messmaschine vermessen und die Messdaten werden in eine die Messdaten verarbeitenden Steuereinrichtung eingelesen. Aus einem gesinterten Material der Zusammensetzung Zr02-TZP mit einem Zr02-Hf02-Anteil von 94.8 bis 95.3 Gew.%, Y203 Gehalt von 4.8 - 5.2 Gew.%, und einer maximalen Konzentration von Drittoxiden (Verunreinigungen) von kleiner als 0.1% mit nachgewiesener Biokompatibilität, einem monoklinen Anteil von unter 5% im Volumen, und einen Radioaktivitätslevel von unter 10 Bq pro kg, entsprechend max. 0.03 Mikro-Sievert/Jahr Strahlenbelastung, wird ein Halbzeug in Form einer Platte der Grösse 20 × 10 × 32 mm hergestellt. Aus dieser Platte wird unter Benutzung einer von der Steuereinrichtung in drei Richtungen gesteuerten Bearbeitungsmaschine die Form der proximalen Phalanx herausgeschliffen. Dazu werden Werkzeuge mit metallisch gebundenen Diamantkörnern mit 6mm Durchmesser eingesetzt. Die gewählte Umdrehungszahl beträgt 22'000 rpm, die Oberflächengeschwindigkeit 0.69 m/sec. Die Zustellung beträgt 0.2 mm/min, der Vorschub 07 cm/sec.

Nach Beendigung des Bearbeitungsprozesses wird die proximale Phalanx im Bereich der Gelenkfläche durch mehrstufiges Polieren auf eine Rauhtiefe von besser als Ra = 0.08 µm gebracht.

### Beispiel II: Mittlerer Phalanx

Ein Kunststoffmodell eines Einsatzes für ein Fingergelenk (mittlerer Phalanx) wird mit Hilfe einer 3D-Messmaschine vermessen und die Messdaten werden in eine die Messdaten verarbeitende Steuereinrichtung eingelesen. Aus einem Material der Zusammensetzung Zr02-TZP mit einem Zr02-Hf02-Anteil von 94,5 - 95,3 Gew.%, Y203 Gehalt von 4,8 - 5,2 Gew.% und einer maximalen Konzentration von Drittoxiden (Verunreinigungen) von kleiner als 0,1%, mit nachgewiesener Biokompatibilität, einem monoklinen Anteil von unter 5% im Volumen und einem Radioaktivitätslevel von unter 10 Bq pro kg, entsprechend max. 0.03 Mikro-Sievert/Jahr Strahlenbelastung wird ein Halbzeug in Form einer Platte der Grösse 15 × 10 × 28 mm hergestellt. Aus dieser Platte wird unter Benutzung einer von der Steuereinrichtung in drei Richtungen gesteuerten Bearbeitungsmaschine die Form der mittleren Phalanx herausgeschliffen. Dazu werden Werkzeuge mit metallisch gebundenen Diamantkörnern mit einem Durchmesser von 5mm eingesetzt. Die gewählte Umdrehungszahl beträgt 28.000 Umdrehungen pro Minute, die Oberflächengeschwindigkeit 0,73 Meter pro Sekunde, die Zustellung beträgt 0,3 Millimeter pro Minute und der Vorschub 0,85 Zentimeter pro Sekunde. Nach Beendigung des maschinellen Bearbeitungsvorganges wird die Gleitfläche der mittleren Phalanx auf eine Rauhtiefe besser als 0,05 µm gebracht.

### Beispiel III Kniegelenk

Aus einem um 30% linear vergrösserten dreidimensionalen Metall-Modell eines linken femoralen Kniegelenk-Teiles wird eine Gipsform hergestellt (Abdruck). Mit Hilfe dieser Form wird im Verfahren des Schlickergiessens oder eines äquivalenten Formgebungsverfahrens ein keramischer Rohling hergestellt. Die Zusammensetzung des verwendeten Rohstoffes ist: Zr02 + Hf02 94.9 Gew.%, Y203 = 5.02%, Verunreinigungen = 0.08%. Der Keramikrohling wird getrocknet und bei 1465° gebrannt. Nach dem Brennen wir er einer HIP (heissisostatisches Nachverdichten) Behandlung bei 900 bar Druck, T = 1375°C, Dauer = 1 Stunde, Argon Atmosphäre, unterzogen. Das Material wird oxidierend bei 1100°C, 1 Stunde, nachbehandelt. Die resultierende weisse Keramik ist ausgezeichnet durch eine nachgewiesene Biokompatibilität, einen monoklinen Anteil von unter 5 Vol. % und einen Radioaktivitätslevel von unter 10 Bq/kg, entsprechend einer Strahlenbelastung von max. 0.03 Mikrosievert pro Jahr (gemittelt über 50 Jahre).

Das Halbzeug mit den Abmessungen von 82.6 × 66.4 × 63.5 mm wird in einem rechteckigen Halter mittels einer Aufnahme fixiert. Mittels einer Bearbeitungsmaschine wird die Aussenform des femoralen Kniegelenkes in zwei Stufen mittels CIM (Computer Integrated Manufacturing)-methoden bearbeitet. Dazu finden metallgebundene Diamantwerkzeuge (Schleifstifte) des Durchmessers 12 und 14.5 mm mit Hohlstruktur und Hartmetallhalterung Verwendung. Die gewählte Körnung ist abgestuft von D 91 über D 126 bis D 151.

Die Umdrehungszahl beträgt 45'000 rpm, die Oberflächengeschwindigkeit 2.8 m/sec. Der Vorschub beträgt 0.8 cm/sec., die Zustellung 0.4 mm/min

Die artikulierten Gelenkoberflächen werden anschliessend mittels vibrierender Polierstufe und Diamantpasten konturangepasst auf eine Oberflächengüte von besser als 0.09 µm (Ra) poliert.

### Beispiel IV: Halswirbelstabilisator

Ein Rohling der Abmessung 18 × 26 - 94 mm und einer Dicke von 0.5 - 1,5 mm wird aus ZR02-TZP-Rohstoff der Zusammensetzung gemäss Beispiel III mittels eines geeigneten Presswerkzeuges axial gepresst. Die Platte weist in der Längsrichtung eine zylindrische Krümmung entsprechend einem Radius von 50 - 250 mm, vorzugsweise 150 mm auf.

Der Rohling wird in oxidierender Atmosphäre gebrannt bei Bedingungen wie in Beispielen I-III. Nach der Sinterung wird das plattenförmige Halbzeug in einen Halter (Aufnahme) eingespannt und nach einem vorher eingegebenen Programm mittels CIM (Dreidimensional Computer Integrated Manufacturing) 3D-bearbeitet. Dabei werden die Aussenkonturen mittels Diamantfräsen, die inneren Oeffnungen durch Bohren bzw. Fräsen mittels geeigneter Diamantwerke bearbeitet. Die Bearbeitungsbedingungen sind wie folgt:
- Werkzeug Durchmesser 4 mm
- Umdrehungszahl 50'000 rpm
- Oberflächengeschwindigkeit 8.3 m/sec.
- Diamantkörnung D 126
- Vorschub 1.5 cm/sec.
- Zustellung 0.6 mm/min.

Nach der Bearbeitung der Konturen wird das Werkstück einer Oberflächenglättung durch Gleitschleifen unterzogen, wobei die Gleitschleifkörper aus 92% AI203 bestehen. Die erreichbare Oberflächengüte liegt bei Ra = 0.86 µm.

## Patentansprüche

1. Verfahren zur Herstelleung hochpräziser und dreidimensional komplexer keramischer Endo- und Exoprothesen, bestehend aus
(a) Formung eines porösen, keramischen Rohlings folgender Zusammensetzung (in Gewichtsprozent): Zirkonoxid und Hafniumoxid 94,8% bis 95,3 %, Yttriumoxid 4,% bis 5,2%, Drittoxide kleiner als 0,1% mit einem monoklinen Anteil unter 5 Volumen% und Umarbeiten dieses Rohlings durch Dichtsintern oder Infiltration zu einem Halbzeug,
(b) Bearbeiten dieses Halbzeuges auf Endmass einer Prothese mittels eines rotierenden Werkzeuges aus metallisch gebundenen Diamantkörnern mit Drehzahlen von 10'000 bis 50'000 Umdrehungen pro Minute, Zustellungen von 0,1 bis 0,7 Millimetern pro Minute, Vorschubgeschwindigkeiten von 0,3 bis 3,0 cm pro Sekunde und Oberflächengeschwindigkeiten für das Werkzeug von 0,5 bis 9,0 Meter pro Sekunde.

2. Verfahren nach Anspruch 1, **dadurch gekennzeichnet, dass** der Rohling als Rondelle ausgebildet ist.

3. Verfahren nach Anspruch 1, **dadurch gekennzeichnet, dass** der Rohling durch Schlickergiessen gebildet, getrocknet, gebrannt, heissisostatisch nachverdichtet und anschliessend oxidierend nachbehandelt und zu einem Halbzeug verarbeitet wird.

4. Verfahren nach einem der Ansprüche 1 bis 3, **dadurch gekennzeichnet, dass** die Umarbeitung zu einer Prothese vermittels digitaler Erfassung und Berechnung der Masse eines Prothesenmodells, Eingabe der Erfassungs- und Berechnungsdaten in eine datenverarbeitende Steuereinrichtung und Ausgabe der Daten an eine in drei Koordinaten bewegbare Bearbeitungseinrichtung zur Bewegung des Werkzeuges erfolgt.

## Claims

1. A process for the production of highly accurate and three dimensionally complex ceramic endo- and exoprostheses comprising
(a) forming a porous ceramic blank of the following composition (in weight percent) zirconium oxide and hafnium oxide 94,8% up to 95,3%, yttrium oxide 4% up to 5,2%, any further oxide less than 0,1% with a monoclinic proportion of below 5% working over this blank by means of dense sintering or filtration to form a semi-finished product,
(b) working over the semi-finished product to final dimensions of a prosthesis by means of a rotating tool comprising metallically bound diamond grains at a speed of rotation of between 10'000 and 50'000 revolutions per minute, an infeed rate of between 0,1 and 0,7 millimeters per minute, a feed rate along the workpiece of between 0,3 and 3.0 centimeters per second and surface speeds of the tool of between 0,5 and 9.0 meters per second.

2. A process according to claim 1 **characterised** thereby that the blank is shaped as a disk.

3. A process according to claim 1 **characterised** thereby that the blank is formed by way of slip casting, dried, roasted, subjected to hot isostatic post-compacting, and then subjected to oxidizing posttreatment to form the semi-finished product.

4. A process according to one of the claims 1 to 3 **characterised** thereby that the working over operation to form a prosthesis is effected by means of digital detection and calculation of the dimensions of a prosthesis pattern input of the detection and calculation data into a data processing control means and output of the data to a machining means movable in three co-ordinates for moving the tool

## Revendications

1. Procédé de fabrication d'endoprothèses et d'exoprothèses en céramique, de grande précision et aux formes tridimensionnelles complexes, comprenant
a) formage d'une ébauche en céramique poreuse avec la composition suivante (en pourcentage en poids) : oxyde de zirconium et oxyde de hafnium 94,8 % à 95,3 %, oxyde d'yttrium 4 % à 5,2 %, oxydes tiers inférieurs à 0,1 % avec une teneur monoclinique inférieure à 5 % en volume et transformation de cette ébauche par frittage à densité maximale ou infiltration en vue d'obtenir un demi-produit,
b) usinage de ce demi-produit aux dimensions finales d'une prothèse au moyen d'un outil rotatif avec des grains de diamant liés métalliquement, avec des vitesses de rotation de 10 000 à 50 000 tours par minute, des vitesses d'approche de 0,1 à 0,7 millimètre par minute, des vitesses d'avance de 0,3 à 3,0 cm par seconde et des vitesses superficielles pour l'outil de 0,5 à 9,0 mètres par seconde.

2. Procédé selon la revendication 1 , **caractérisé en ce que** l'ébauche est réalisée sous forme de rondelle.

3. Procédé selon la revendication 1, **caractérisé en ce que** l'ébauche est formée par coulée en barbotine, séchage, cuisson, post-compactage isostatique à chaud et, ensuite, par traitement postérieur par voie oxyde, et est transformée pour obtenir un demi-produit.

4. Procédé selon l'une quelconque des revendications 1 à 3, **caractérisé en ce que** la transformation pour obtenir une prothèse est effectuée au moyen d'un enregistrement et calcul numériques de la masse d'un modèle de prothèse, la saisie des données d'enregistrement et de calcul dans une unité de commande assistée par ordinateur et édition des données vers une unité d'usinage mobile dans trois directions de coordonnées, destinée à déplacer l'outil.
